(19) Europäisches Patentamt
European Patent Office
Office européen des brevets

(11) EP 3 636 245 A1

(12) EUROPEAN PATENT APPLICATION
published in accordance with Art. 153(4) EPC

(43) Date of publication:
15.04.2020 Bulletin 2020/16

(21) Application number: 18812709.6

(22) Date of filing: 06.06.2018

(51) Int Cl.:
*A61K 8/37* (2006.01) *A61K 8/06* (2006.01)
*A61K 8/25* (2006.01) *A61K 8/35* (2006.01)
*A61K 8/36* (2006.01) *A61K 8/40* (2006.01)
*A61K 8/41* (2006.01) *A61K 8/60* (2006.01)
*A61K 8/73* (2006.01) *A61K 8/81* (2006.01)
*A61K 8/895* (2006.01) *A61K 8/92* (2006.01)
*A61Q 17/04* (2006.01)

(86) International application number:
PCT/JP2018/021662

(87) International publication number:
WO 2018/225768 (13.12.2018 Gazette 2018/50)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 08.06.2017 JP 2017113231

(71) Applicant: Shiseido Company, Ltd.
Tokyo 104-0061 (JP)

(72) Inventors:
- TOUATI, Marianne Ayaka
  Yokohama-shi
  Kanagawa 224-8558 (JP)
- YAMAKI, Satoshi
  Yokohama-shi
  Kanagawa 224-8558 (JP)
- WATANABE, Yurika
  Yokohama-shi
  Kanagawa 224-8558 (JP)
- KATORI, Takahiro
  Yokohama-shi
  Kanagawa 224-8558 (JP)

(74) Representative: Ter Meer Steinmeister & Partner
Patentanwälte mbB
Nymphenburger Straße 4
80335 München (DE)

# (54) WATER-IN-OIL EMULSIFIED COSMETIC PREPARATION

(57) The present invention provides a water-in-oil emulsion cosmetic that can obtain high ultraviolet protection effects even without substantially blending an ultraviolet scattering agent therein. The water-in-oil emulsion cosmetic of the present invention is characterized by containing (A) 6 to 40% by mass of an ultraviolet absorbing agent; (B) 15% by mass or more of a non-volatile polar oil having ester bonds (except that the (A) ultraviolet absorbing agent is not included); and (C) 0.5 to 15% by mass of an oil phase thickener. It is particularly preferable for the present invention to further contain (D) a powder having an average particle size of 15 μm or smaller.

EP 3 636 245 A1

## Description

TECHNICAL FIELD

The present invention relates to a water-in-oil emulsion cosmetic. More specifically, the present invention relates to a water-in-oil emulsion cosmetic in which the ultraviolet protection effects are markedly improved by blending prescribed amounts of a non-volatile polar oil and an oil phase thickener.

BACKGROUND ART

Protecting the skin from damage due to ultraviolet rays is one of the important problems in skin care and body care, and various types of UV-care cosmetics have been developed in order to minimize the detrimental impact of ultraviolet rays on skin. Sunscreen cosmetics, which are a type of UV-care cosmetic, are cosmetics that are intended to protect the skin from damage due to ultraviolet rays by covering the skin with a coating film containing an ultraviolet absorbing agent or an ultraviolet scattering agent, thereby absorbing or scattering UVA and UVB rays and suppressing the amount of ultraviolet radiation that reaches the skin (Non-Patent Document 1).

In general, sunscreen cosmetics are commonly obtained by blending an appropriate combination of ultraviolet absorbing agents that chemically absorb ultraviolet rays and ultraviolet scattering agents that physically reflect/scatter ultraviolet rays, in order to obtain a high SPF (Sun Protection Factor) value. However, ultraviolet scattering agents, represented by titanium oxide and zinc oxide, scatter visible light in addition to ultraviolet rays. Thus, when applied to skin, so-called "unnatural whiteness" occurs, and there is a tendency for the appearance to be noticeably white. Additionally, there is the problem that, when there is a high content of such agents having hydrophobically treated surfaces, the washability becomes poor. Furthermore, the astringent effect of zinc oxide imparts a feeling of dryness to the skin and may cause the feeling in use to become worse.

Therefore, there have been attempts at obtaining a high SPF without blending in an ultraviolet scattering agent, by blending a large amount of an ultraviolet absorbing agent instead. For example, Patent Document 1 proposes not blending in an ultraviolet scattering agent and blending a large amount of an ultraviolet absorbing agent, and meanwhile, blending in 0.5 to 2% by mass of a dextrin fatty acid ester and/or a sucrose fatty acid ester relative to the overall amount of the cosmetic in order to suppress oiliness and stickiness that occurs when increasing the amount of the ultraviolet absorbing agent.

However, it is difficult to compensate for the absence of an ultraviolet scattering agent solely by blending in a large amount of an ultraviolet absorbing agent, and improvements in the ultraviolet protection effects are still sought.

RELATED ART

PATENT DOCUMENTS

Patent Document 1: JP 2011-126832 A

NON-PATENT DOCUMENTS

Non-Patent Document 1: Shin-keshohin-gaku [New Cosmetology], 2nd edition, edited by Takeo Mitsui, 2001, published by Nanzando, pp. 497-504

SUMMARY OF THE INVENTION

PROBLEM TO BE SOLVED BY THE INVENTION

A purpose of the present invention is to provide a water-in-oil emulsion cosmetic that is able to provide sufficiently high ultraviolet protection effects even without substantially blending in an ultraviolet scattering agent.

MEANS FOR SOLVING THE PROBLEM

The present inventors performed diligent investigations towards solving the aforementioned problem, as a result of which they discovered that high ultraviolet protection effects can be obtained, even when reducing the blended amount or not blending in an ultraviolet scattering agent, by using an ultraviolet absorbing agent in combination with a prescribed amount of a non-volatile polar oil and an oil phase thickener, thereby completing the present invention.

In other words, the present invention provides a water-in-oil emulsion cosmetic containing:

(A) 6 to 40% by mass of an ultraviolet absorbing agent;
(B) 15% by mass or more of a non-volatile polar oil having ester bonds (except that the (A) ultraviolet absorbing agent is not included); and
(C) 0.5 to 15% by mass of an oil phase thickener.

EFFECTS OF THE INVENTION

Due to the above-mentioned features, the present invention is able to obtain excellent ultraviolet protection effects, even when substantially no ultraviolet scattering agents are blended therein. For this reason, a cosmetic with excellent versatility that does not result in unnatural whiteness can be provided.

MODES FOR CARRYING OUT THE INVENTION

As mentioned above, the water-in-oil emulsion cosmetic of the present invention is characterized by containing (A) an ultraviolet absorbing agent, (B) a non-volatile polar oil, and (C) an oil phase thickener. The respective components constituting the cosmetic of the present invention will be explained in detail below.

<(A) Ultraviolet absorbing agent>

As the (A) ultraviolet absorbing agent (hereinafter sometimes referred to simply as "component (A)") blended in the water-in-oil emulsion cosmetic according to the present invention, it is possible to use one that is normally blended into sunscreen cosmetics.

The (A) ultraviolet absorbing agent is not particularly limited, but specific examples include organic ultraviolet absorbing agents such as ethylhexyl methoxycinnamate, octocrylene, 2-hydroxy-4-methoxybenzophenone, dimethicodiethylbenzalmalonate, t-butyl methoxydibenzoylmethane, ethylhexyl triazone, diethylamino hydroxybenzoyl hexyl benzoate, bis-ethylhexyloxyphenol methoxyphenyl triazine, methylene bis-benzotriazolyl tetramethylbutylphenol, phenylbenzimidazole sulfonic acid, homosalate and ethylhexyl salicylate.

The blended amount of component (A) should be 6 to 40% by mass, more preferably 8 to 35% by mass, and even more preferably 10 to 30% by mass relative to the overall amount of the water-in-oil emulsion cosmetic. If the blended amount of component (A) is less than 6% by mass, then sufficient ultraviolet protection effects cannot be obtained, and even if more than 40% by mass is blended, an increase in the ultraviolet protection effects that is commensurate with the blended amount cannot be expected, and this is undesirable for making the stability worse and the like. Component (A) may be used as one type alone, or may be a combination of two or more types.

<(B) Non-volatile polar oil>

The (B) non-volatile polar oil (hereinafter sometimes referred to simply as "component (B)") blended into the water-in-oil emulsion cosmetic according to the present invention is a polar ester oil that does not exhibit volatility at ambient temperature (25 °C) and ambient pressure (1 atm ($9.8 \times 10^4$ Pa)) (for example, including oils having a boiling point equal to or higher than approximately 200 °C at ambient pressure), and that has fluidity at ambient temperature and ambient pressure.

In this case, the "polar oil" is not particularly limited as long as it is an oil having high polarity among oils that are generally used in cosmetics. For example, an oil having a relative permittivity of approximately 5 or higher, preferably approximately 10 or higher, is preferably used.

While the above-mentioned (A) ultraviolet absorbing agent includes some that have ester bonds and that are non-volatile and polar, such ultraviolet absorbing agents are considered to be the component (A) in the present invention and are not included in component (B).

Specific examples of the (B) non-volatile polar oil include diisopropyl sebacate, isopropyl myristate, glyceryl tri-2-ethylhexanoate, cetyl 2-ethylhexanoate, tripropylene glycol dineopentanoate, isononyl isononanoate, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, lanolin acetate, isocetyl stearate, isocetyl isostearate, cholesteryl 12-hydroxystearate, ethylene glycol di-2-ethylhexanoate, dipentaerythritol fatty acid ester, N-alkylglycol monoisostearate, neopentylglycol dicaprate, diisostearyl malate, glyceryl di-2-heptylundecanoate, trimethylolpropane tri-2-ethylhexanoate, trimethylolpropane triisostearate, pentraerythrityl tetra-2-ethylhexanoate, triethylhexanoin (glyceryl tri-2-ethylhexanoate), glyceryl trioctanoate, glyceryl triisopalmitate, trimethylolpropane triisostearate, cetyl 2-ethylhexanoate, 2-ethylhexyl palmitate, glyceryl trimyristate, tri-2-heptylundecanoic acid glyceride, castor oil fatty acid methyl ester, oleyl oleate, acetoglyceride, 2-heptylundecyl palmitate, diisobutyl adipate, N-lauroyl-L-glutamic acid-2-octyldecyl ester, di-2-heptylundecyl adipate, ethyl laurate, di-2-ethylhexyl sebacate, 2-hexyldecyl myristate, 2-hexyldecyl palmitate, 2-hexyldecyl

adipate, 2-ethylhexyl succinate, triethyl citrate, olive oil, sunflower oil, camellia oil, soybean oil, jojoba oil, macadamia nut oil, meadowfoam oil and the like.

The blended amount of component (B) should be 15% by mass or more, more preferably 18% by mass or more, and even more preferably 20% by mass or more relative to the overall amount of the water-in-oil emulsion cosmetic. If the blended amount of component (B) is less than 15% by mass, then the ultraviolet protection effects cannot be sufficiently improved. On the other hand, if a large amount of component (B) is blended, then there is a tendency for the feeling in use and the water resistance to become worse. Thus, at most, the blended amount should be 70% by mass or less, and should more preferably be 50% by mass or less. Component (B) may be used as one type alone or as a combination of two or more types.

<(C) Oil phase thickener>

The (C) oil phase thickener (hereinafter sometimes referred to simply as "ingredient (C)") blended in the water-in-oil emulsion cosmetic according to the present invention is a substance that can adjust the viscosity of the oil phase in the water-in-oil emulsion cosmetic. As component (C), dextrin fatty acid esters, sucrose fatty acid esters, fatty acids or salts thereof, hardened vegetable oils, solid or semi-solid vegetable oils, organically modified clay minerals, glyceryl fatty acid esters and amino acid-based gelling agents are preferred.

Dextrin fatty acid esters are esters of dextrin or reduced dextrin with a higher fatty acid, which may be used without any particular restrictions as long as they are generally used in cosmetics. As the dextrin or reduced dextrin, one in which the average degree of sugar polymerization is 3 to 100 is preferably used. Additionally, as the constituent fatty acids in the dextrin fatty acid ester, a saturated fatty acid having 8 to 22 carbon atoms is preferably used. Specific examples include dextrin palmitate, dextrin oleate, dextrin stearate, dextrin myristate, dextrin (palmitate/2-ethylhexanoate) and the like.

As the sucrose fatty acid ester, one in which the fatty acid is linear or branched, saturated or unsaturated, and having 12 to 22 carbon atoms is preferably used. Specific examples include sucrose caprylic acid esters, sucrose capric acid esters, sucrose lauric acid esters, sucrose myristic acid esters, sucrose palmitic acid esters, sucrose stearic acid esters, sucrose oleic acid esters, sucrose erucic acid esters and the like.

The fatty acid may be solid at ambient temperature, and examples include myristic acid, palmitic acid, stearic acid, behenic acid and the like. Additionally, the fatty acid salt may be a calcium salt, a magnesium salt, an aluminum salt or the like of the above.

Examples of the hardened vegetable oil include hardened palm kernel oil, hardened castor oil, hydrogenated peanut oil, hydrogenated rapeseed oil, hydrogenated palm oil, hydrogenated camellia oil, hydrogenated soybean oil, hydrogenated olive oil, hydrogenated macadamia nut oil, hydrogenated sunflower oil, hydrogenated wheat germ oil, hydrogenated rice germ oil, hydrogenated rice bran oil, hydrogenated cottonseed oil, hydrogenated avocado oil and the like.

Additionally, like the hardened vegetable oils, it is also possible to use a vegetable oil that is solid or semi-solid at room temperature. In this case, a solid oil refers to an oil that is solid at 25 °C, and a semi-solid oil refers to an oil in which half the amount thereof is solid at 25 °C. More specifically, one in which the melting point is within the range from 44 °C to 90 °C, the viscosity measured with a B-type viscometer at 25 °C is 5000 mPa●s or higher, or furthermore, 10,000 mPa●s or higher, is preferred. Examples of vegetable oils that are solid or semi-solid at room temperature include cacao butter, coconut oil, palm oil, palm kernel oil, Japan tallow, shea butter and the like.

As the organically modified clay mineral, it is possible to use a clay mineral modified by a quaternary ammonium salt type cationic surfactant, represented by the following general formula (1), which is a type of colloidal hydrated aluminum silicate having a three-layered structure.

$$(X,Y)_{2-3}(Si,Al)_4O_{10}(OH)_2Z_{1/3}\cdot nH_2O \qquad (1)$$

where X = Al, Fe(III), Mn(III) or Cr(III); Y = Mg, Fe(II), Ni, Zn or Li; and Z = K, Na or Ca.

Specifically, the organically modified clay mineral can be obtained by treating, with a quaternary ammonium salt type cationic surfactant, a clay mineral which may be a natural or synthetic (in this case, an (OH) group in the formula is substituted with a fluorine) clay mineral in the montmorillonite group, such as montmorillonite, saponite or hectorite (commercial products include Veegum, Kunipia, Laponite, etc.), or a synthetic mica known under the name of sodium silicic mica or sodium or lithium taeniolite (commercial products include Dimonite, manufactured by Topy Industries, Ltd. etc.).

The quaternary ammonium salt type cationic surfactant used in this case is represented by the following general formula (2):

[Chemical Formula 1]

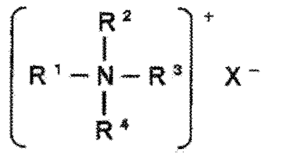 ··· (2)

where $R^1$ represents an alkyl group having 10 to 22 carbon atoms or a benzyl group, $R^2$ represents a methyl group or an alkyl group having 10 to 22 carbon atoms, $R^3$ and $R^4$ represent alkyl groups or hydroxyalkyl groups having 1 to 3 carbon atoms, and X represents a halogen atom or a methylsulfate residue.

Examples of the quaternary ammonium salt type cationic surfactant include dodecyltrimethylammonium chloride, myristyltrimethylammonium chloride, cetyltrimethylammonium chloride, stearyltrimethylammonium chloride, arachyltrimethylammonium chloride, behenyltrimethylammonium chloride, myristyldimethylethylammonium chloride, cetyldimethylethylammonium chloride, stearyldimethylethylammonium chloride, arachyldimethylethylammonium chloride, behenyldimethylethylammonium chloride, myristyldiethylmethylammonium chloride, cetyldiethylmethylammonium chloride, stearyldiethylmethylammonium chloride, arachyldiethylmethylammonium chloride, behenyldiethylmethylammonium chloride, benzyldimethylmyristylammonium chloride, benzyldimethylcetylammonium chloride, benzyldimethylstearylammonium chloride, benzyldimethylbehenylammonium chloride, benzylmethylethylcetylammonium chloride, benzylmethylethylstearylammonium chloride, dibehenyldihydroxyethylammonium chloride, and corresponding bromides and the like, and further thereto, dipalmitylpropylethylammonium methylsulfate and the like. When carrying out the present invention, one or more of these compounds may be arbitrarily selected.

Representative examples of organically modified clay minerals include dimethyl distearyl ammonium hectorite (distearyldimonium hectorite), dimethylalkylammonium hectorite, benzyldimethylstearylammonium hectorite, distearyldimethylammonium chloride-treated aluminum-magnesium silicate and the like. Of these, dimethyldistearylammonium hectorite is particularly preferred. As commercial products, Bentone 27 (benzyldimethylstearylammonium chloride-treated hectorite, manufactured by Elementis Japan KK) and Bentone 38 (distearyldimethylammonium chloride-treated hectorite, manufactured by Elementis Japan KK) are preferred.

Glyceryl fatty acid esters are esterified reaction products obtained by reacting glycerin, a diprotic acid having 18 to 28 carbon atoms, and a fatty acid having 8 to 28 carbon atoms (excluding diprotic acids). They may be used without any particular restrictions as long as they are generally used in cosmetics. Specific examples include glyceryl (behenate/isostearate/eicosanedioate), glyceryl (behenate/eicosanedioate) and polyglyceryl-10 (behenate/eicosanedioate), etc.

Examples of amino acid-based gelling agents include dibutyl lauroyl glutamide, dibutyl ethylhexanoyl glutamide, polyamide-8, polyamide-3, N-lauroyl-L-glutamic acid dibutyl amide and the like.

The blended amount of component (C) should preferably be 0.5 to 15% by mass, more preferably 1 to 10% by mass, and even more preferably 1 to 8% by mass relative to the overall amount of the water-in-oil emulsion cosmetic. If the blended amount of component (C) is less than 0.5% by mass, then the ultraviolet protection effects cannot be sufficiently improved, and if more than 15% by mass is blended, then the viscosity becomes high, and this is undesirable in terms of the properties when used, such as by becoming difficult to spread over the skin. Additionally, component (C) may be one type used alone or may be a combination of two or more types. In particular, it is preferable for two or more types of oil phase thickeners to be blended, and for one of the types to be an organically modified clay mineral. Furthermore, it is preferable to blend 2% by mass or more of a (C) oil phase thickener other than an organically modified clay mineral.

<(D) Powder having average particle size of 15 μm or smaller>

In the water-in-oil emulsion cosmetic of the present invention, the ultraviolet protection effects can be improved by containing the above-mentioned components (A) to (C).

However, in order to further improve the protection effects against ultraviolet rays, it is preferable to further blend (D) a powder having an average particle size of 15 μm or smaller (hereinafter sometimes referred to simply as "component (D)") in addition to the above-mentioned components (A) to (C). By blending in component (D), it is possible to obtain ultraviolet protection effects that are equivalent to or even higher than those when blending in an ultraviolet scattering agent.

Component (D) has an average particle size of 15 μm or smaller, more preferably 12 μm or smaller. Although the lower limit of the average particle size is not particularly limited, it should preferably be 3 μm or larger for the reason that it becomes more difficult to handle and the like. The average particle size can be measured by means of a laser diffraction/scattering method.

Component (D) used in the present invention is preferably selected from among the following types (i) to (iv):

(i) talc;
(ii) silicone powder;
(iii) crosslinked poly(methyl methacrylate) powder; and
(iv) silica.

(i) Talc

Talc is a silicic acid salt-based clay mineral, which is widely used in cosmetics as an extender pigment in the same way as mica, sericite and the like.
In the present invention, hydrophobically treated talc, wherein talc is the base material and the surface thereof is hydrophobically treated, is particularly preferred.
The hydrophobic treatment is not particularly restricted, but may, for example, be a silicone treatment (treatment with a silicone oil such as methylhydrogen polysiloxane, dimethyl polysiloxane or methylphenyl polysiloxane; an alkylsilane such as methyltrimethoxysilane, ethyltrimethoxysilane, hexyltrimethoxysilane or octyltrimethoxysilane; or a fluoroalkylsilane such as trifluoromethylethyl trimethoxysilane or heptadecafluorodecyl trimethoxysilane), a fatty acid treatment (treatment with palmitic acid, isostearic acid, stearic acid, lauric acid, myristic acid, behenic acid, oleic acid, rosin acid, 12-hydroxystearic acid or the like), a fatty acid soap treatment (treatment with aluminum stearate, calcium stearate, 12-hydroxystearic acid or the like), or a fatty acid ester treatment (treatment with a dextrin fatty acid ester, a cholesterol fatty acid ester, a sucrose fatty acid ester, a starch fatty acid ester or the like). These hydrophobic treatments may be performed in accordance with conventional methods. Among hydrophobic treatments, silicone treatments are suitable for being able to impart high stability to the powder particles and the like.
As the hydrophobically treated talc, it is possible to use a commercial product, an example of which is "Calcium stearate-treated talc" (Fujimoto Chemicals Co., Ltd.).

(ii) Silicone powder

As the silicone powder, one that is normally used as a cosmetic raw material may be used. As examples of silicone powders, methylpolysiloxane network polymers, crosslinked methylpolysiloxane, crosslinked silicone-network silicone block copolymers, silylated silica and the like are known. Of these, crosslinked silicone-network silicone block copolymers are particularly preferred.
An example of a silicone powder commercial product that can be used is "KSP-100" (manufactured by Shin-etsu Chemical Co., Ltd. (vinyl dimethicone/methicone silsesquioxane) crosspolymer, average particle size 5 μm).

(iii) Crosslinked poly(methyl methacrylate) powder

As the crosslinked poly(methyl methacrylate) powder (PMMA powder), any type that is normally used as a cosmetic raw material may be used, as long as the average particle size is 15 μm or smaller.
As the crosslinked poly(methyl methacrylate) powder, it is possible to use a commercial product, an example of which is "Ganzpearl" (manufactured by Aica Kogyo Co., Ltd., average particle size 8 μm).

(iv) Silica

As the silica (silicic anhydride), any type that is normally used as a cosmetic raw material may be used, regardless of whether it is porous or non-porous. Examples of commercial products include "Sunsphere H-31" (manufactured by AGC Si-Tech Co., Ltd.) (average particle size 3 μm), "Sunsphere H-51" (manufactured by AGC Si-Tech Co., Ltd.) (average particle size 5 μm) and "Sunsphere H-121" (manufactured by AGC Si-Tech Co., Ltd.) (average particle size 12 μm). Additionally, it is also possible to use silica having a hydrophobically treated surface such as, for example, dimethylsilylated silicic anhydride and trimethylsilylated silicic anhydride.
The component (D) selected from the above-mentioned (i) to (iv) may be of one type used alone, or may be a combination of two or more types. The blended amount of component (D) is not particularly limited, but should preferably be 1 to 30% by mass, more preferably 1 to 25% by mass, and even more preferably 1 to 20% by mass relative to the overall amount of the water-in-oil emulsion cosmetic. If the blended amount of component (D) is less than 1% by mass, then there is a tendency for the effects of component (D) to not be able to be sufficiently obtained. If more than 30% by mass is blended, then there are cases in which the properties when used become worse, such as squeakiness, smudges and stickiness occurring, and cases in which the cosmetic becomes difficult to formulate.

<Ultraviolet scattering agent>

The water-in-oil emulsion cosmetic of the present invention is able to realize sufficient ultraviolet protection effects by containing the above-mentioned components (A) to (C), even without blending in an ultraviolet scattering agent.

However, it is permissible to blend in a small amount of an ultraviolet scattering agent for the purpose of further improving the protection effects against ultraviolet rays. When blending an ultraviolet scattering agent into the water-in-oil emulsion cosmetic of the present invention, it should be 6% by mass or less, preferably 5% by mass or less, more preferably 4% by mass or less, and particularly preferably 3% by mass or less relative to the overall amount of the water-in-oil emulsion cosmetic in order to suppress unnatural whiteness and reductions in the texture due to the ultraviolet scattering agent.

The ultraviolet scattering agent that may be blended in the water-in-oil emulsion cosmetic of the present invention is not particularly limited, and an ultraviolet scattering agent that is normally used in cosmetics may be used. Examples of ultraviolet scattering agents include fine-particle metal oxides such as zinc oxide, titanium oxide, iron oxide, cerium oxide and tungsten oxide, and these metal oxides with surfaces that have been subjected to various hydrophobic surface treatments. As the hydrophobic surface treatment agent, it is possible to use those that are generally used in the cosmetic field including, for example, silicones such as dimethicone and alkyl-modified silicone, alkoxysilanes such as octyltriethoxysilane, dextrin fatty acid esters such as dextrin palmitate, and fatty acids such as stearic acid.

Aside from the above-mentioned essential components, the water-in-oil emulsion cosmetic of the present invention may appropriately contain, as needed, components that are normally used in cosmetics such as, for example, oils, water, alcohols, surfactants, oil-based active agents, water-based active agents, water phase thickeners, humectants and antioxidants.

Among the above, a surfactant that may be used in the present invention is preferably a surfactant having an HLB lower than 8, particularly a silicon-based surfactant, in order to obtain a water-in-oil emulsion state.

Examples of silicone-based surfactants having an HLB lower than 8 include polyoxyalkylene-modified silicones, polyoxyalkylene/alkyl co-modified silicones, polyglycerin-modified silicones and/or polyglycerin/alkyl co-modified silicones. Specific examples include KF-6017 (PEG-10 dimethicone, manufactured by Shin-etsu Chemical Co., Ltd.), KF-6028 (PEG-9 polydimethylsiloxyethyl dimethicone, manufactured by Shin-etsu Chemical Co., Ltd.), ABIL EM 90 (cetyl PEG/PPG-10/1 dimethicone, manufactured by Evonik Goldschmidt Corp.) and KF-6038 (lauryl PEG-9 polydimethylsiloxyethyl dimethicone, manufactured by Shin-etsu Chemical Co., Ltd.), bis-butyl dimethicone polyglyceryl-3 and the like.

The blended amount of the silicone-based surfactant should preferably be 0.1 to 8% by mass, more preferably 0.2 to 7% by mass, even more preferably 0.4 to 5% by mass relative to the overall amount of the water-in-oil emulsion cosmetic.

The water-in-oil emulsion cosmetic of the present invention may be provided not only, for example, as a sunscreen cream, a sunscreen milky lotion or a sunscreen lotion, but may also be used as a foundation, a makeup base, a makeup cosmetic, a hair cosmetic or the like imparted with sunscreen effects, and may be produced by a conventional method.

EXAMPLES

The present invention will be explained in further detail by referring to specific examples below, but the present invention is not limited by the examples indicated below. Additionally, the blended amounts in the following examples and the like are in % by mass unless specially indicated otherwise.

(Examples 1 to 8 and Comparative Examples 1 to 3)

Water-in-oil emulsion cosmetics having the compositions indicated in Tables 1 and 2 below were prepared by heating and melting the oil-based components and dispersing the powder therein, adding the separately mixed water phase thereto, and emulsifying the mixture by stirring.

The cumulative absorbance (Abs) values in the prepared cosmetics were measured by the method indicated below to evaluate the ultraviolet protection effects.

<Measurement of cumulative absorbance (Abs) value>

Cosmetics (samples) according to each example were dripped, in the amount of 2 mg/cm$^2$, onto measurement plates (S plates) (5 $\times$ 5 cm V-grooved PMMA plates, SPFMA STER-PA01), which were coated by finger for 60 seconds, and dried for 15 minutes. Thereafter, the absorbances of the formed coating films were measured by a U-3500 self-recording spectrophotometer manufactured by Hitachi, Ltd. With an uncoated plate as the control, absorbances (Abs) were computed from the expression indicated below, and the measurement values at 280 nm to 400 nm were summed

and assumed to represent the ultraviolet protection effects.

$$Abs = -\log(T/To)$$

T: transmittance of sample, To: transmittance of uncoated plate

[Table 1]

| | Comparative Example 1 | Example 1 | Example 2 |
|---|---|---|---|
| Octocrylene | 3 | 3 | 3 |
| 2-Ethylhexyl para-methoxycinnamate | 8 | 8 | 8 |
| 2-Hydroxy-4-methoxybenzophenone | 3 | 3 | 3 |
| 4-tert-Butyl-4'-methoxydibenzoylmethane | 2.5 | 2.5 | 2.5 |
| Dextrin palmitate | 5 | 5 | 5 |
| Trimethylsiloxysilicic acid | 3 | 3 | 3 |
| Polyoxybutylene (9) polyoxypropylene (1) glycol | 2 | 2 | 2 |
| Isostearic acid | 0.5 | 0.5 | 0.5 |
| Diisopropyl sebacate | 1 | 5 | 7 |
| Glyceryl tri-2-ethylhexanoate | 1 | 5 | 7 |
| Cetyl 2-ethylhexanoate | 1 | 5 | 7 |
| Methyl polysiloxane | 3.5 | 3.5 | 3.5 |
| Dimethyl distearyl ammonium hectorite | 0.5 | 0.5 | 0.5 |
| Decamethylcyclopentasiloxane | 32 | 20 | 14 |
| Lauryl PEG-9 polydimethylsiloxyethyl dimethicone | 1 | 1 | 1 |
| Purified water | bal | bal | bal |
| Table salt | s.a. | s.a. | s.a. |
| Trisodium edetate | s.a. | s.a. | s.a. |
| Glycerin | 1 | 1 | 1 |
| Xylitol | 1 | 1 | 1 |
| Alcohol (*1) | 10 | 10 | 10 |
| Cumulative absorbance (Abs) | 75.3 | 146 | 149.1 |
| (*1) Synthetic alcohol 95% (Japan Alcohol Trading Co., Ltd.) | | | |

[Table 2A]

| | Comparative Example 2 | Example 3 | Example 4 | Example 5 |
|---|---|---|---|---|
| Octocrylene | 3 | 3 | 3 | 3 |
| 2-Ethylhexyl para-methoxycinnamate | 8 | 8 | 8 | 8 |
| 2-Hydroxy-4-methoxybenzophenone | 3 | 3 | 3 | 3 |
| 4-tert-Butyl-4'-methoxydibenzoylmethane | 2.5 | 2.5 | 2.5 | 2.5 |
| Dextrin palmitate | - | 1.5 | 2 | 5 |
| Trimethylsiloxysilicic acid | 3 | 3 | 3 | 3 |
| Polyoxybutylene (9) polyoxypropylene (1) glycol | 2 | 2 | 2 | 2 |

(continued)

| | Comparative Example 2 | Example 3 | Example 4 | Example 5 |
|---|---|---|---|---|
| Isostearic acid | 0.5 | 0.5 | 0.5 | 0.5 |
| Diisopropyl sebacate | 5 | 5 | 5 | 5 |
| Glyceryl tri-2-ethylhexanoate | 5 | 5 | 5 | 5 |
| Cetyl 2-ethylhexanoate | 5 | 5 | 5 | 5 |
| Methyl polysiloxane | 3.5 | 3.5 | 3.5 | 3.5 |
| Decamethylcyclopentasiloxane | 25 | 23.5 | 23 | 20 |
| Lauryl PEG-9 polydimethylsiloxyethyl dimethicone | 1 | 1 | 1 | 1 |
| Purified water | bal | bal | bal | bal |
| Table salt | s.a. | s.a. | s.a. | s.a. |
| Trisodium edetate | s.a. | s.a. | s.a. | s.a. |
| Glycerin | 1 | 1 | 1 | 1 |
| Xylitol | 1 | 1 | 1 | 1 |
| Alcohol (*1) | 10 | 10 | 10 | 10 |
| Cumulative absorbance (Abs) | 88.4 | 90.6 | 93.8 | 117.1 |
| (*1) Synthetic alcohol 95% (Japan Alcohol Trading Co., Ltd.) | | | | |

[Table 2B]

| | Comparative Example 3 | Example 6 | Example 7 | Example 8 |
|---|---|---|---|---|
| Octocrylene | 5 | 5 | 5 | 5 |
| 2-Ethylhexyl para-methoxycinnamate | 3 | 3 | 3 | 3 |
| 4-tert-Butyl-4'-methoxydibenzoylmethane | 2.5 | 2.5 | 2.5 | 2.5 |
| Ethylhexyl triazine | 2 | 2 | 2 | 2 |
| Diethylamino hydroxybenzoyl hexyl benzoate | 2 | 2 | 2 | 2 |
| Polyoxybutylene (9) polyoxypropylene (1) glycol | 2 | 2 | 2 | 2 |
| Isostearic acid | 0.5 | 0.5 | 0.5 | 0.5 |
| Diisopropyl sebacate | 10 | 10 | 10 | 10 |
| Isopropyl myristate | 5 | 5 | 5 | 5 |
| Glyceryl tri-2-ethylhexanoate | 5 | 5 | 5 | 5 |
| Polyamide-8 (*2) | - | 1 | - | - |
| N-Lauroyl-L-glutamic acid dibutyl amide (*3) | - | - | 1 | - |
| Glyceryl (behenate/eicosanedioate) (*4) | - | - | - | 0.5 |
| Trimethylsiloxysilicic acid | 1.5 | 1.5 | 1.5 | 1.5 |
| Methyl polysiloxane | 5 | 5 | 5 | 5 |
| Lauryl PEG-9 polydimethylsiloxyethyl dimethicone | 3 | 3 | 3 | 3 |
| Dimethyl distearyl ammonium hectorite | 0.5 | 0.5 | 0.5 | 0.5 |
| Decamethylcyclopentasiloxane | 12 | 12 | 12 | 12 |
| Calcium stearate-treated talc (average particle size 7 μm) | 5 | 5 | 5 | 5 |

(continued)

| | Comparative Example 3 | Example 6 | Example 7 | Example 8 |
|---|---|---|---|---|
| Crosslinked poly(methyl methacrylate) (average particle size 8 μm), low crosslink density type (crosslink density 10%) | 10 | 10 | 10 | 10 |
| Crosslinked silicone-network silicone block copolymer (average particle size 5 μm) | 1 | 1 | 1 | 1 |
| Purified water | bal | bal | bal | bal |
| Table salt | s.a. | s.a. | s.a. | s.a. |
| Trisodium edetate | s.a. | s.a. | s.a. | s.a. |
| Xylitol | 1 | 1 | 1 | 1 |
| Glycerin | 1 | 1 | 1 | 1 |
| Alcohol (*1) | 6 | 6 | 6 | 6 |
| Cumulative absorbance (Abs) | 157.7 | 166.6 | 168.9 | 170 |
| (*1) Synthetic alcohol 95% (Japan Alcohol Trading Co., Ltd.)<br>(*2) Oleocraft LP-20 (Croda Japan K.K.)<br>(*3) GP-1 (Ajinomoto Healthy Supply Co., Inc.)<br>(*4) Nomucoat HK-G (The Nisshin Oillio Group, Ltd.) | | | | |

As shown in Table 1 above, it was confirmed that the cumulative absorbance (Abs) value is markedly improved by blending 15% by mass or more of the (B) non-volatile polar oil relative to the water-in-oil emulsion cosmetic. Additionally, as shown in Tables 2A and 2B above, it was confirmed that the cumulative absorbance (Abs) value is improved by blending 0.5% by mass or more of the (C) oil phase thickener relative to the water-in-oil emulsion cosmetic.

(Examples 9 and 10 and Comparative Example 4)

The water-in-oil emulsion cosmetics having the compositions indicated in Table 3 below were prepared in the same manner as above, and the ultraviolet protection effects were evaluated by measuring the in-vitro SPF of the cosmetics by the method indicated below.

<Measurement of in-vitro SPF>

Cosmetics (samples) according to each example were dripped, in the amount of 2 mg/cm$^2$, onto measurement plates (S plates) (5 × 5 cm V-grooved PMMA plates, SPFMA STER-PA01), which were coated by finger for 60 seconds, and dried for 15 minutes. Thereafter, the in-vitro SPF was measured with an SPF MASTER (registered trademark) (manufactured by Shiseido Irica Technology Inc.).

[Table 3]

| | Comparative Example 4 | Example 9 | Example 10 |
|---|---|---|---|
| Octocrylene | 3 | 3 | 3 |
| 2-Ethylhexyl para-methoxycinnamate | 8 | 8 | 8 |
| 2-Hydroxy-4-methoxybenzophenone | 2 | 3 | 3 |
| 4-tert-Butyl-4'-methoxydibenzoylmethane | 2.5 | 2.5 | 2.5 |
| Dextrin palmitate | 3 | 5 | - |
| Hydrogenated palm oil | - | - | 2.5 |
| Palm oil | - | - | 1 |

(continued)

| | Comparative Example 4 | Example 9 | Example 10 |
|---|---|---|---|
| Palm kernel oil | - | - | 1.5 |
| Trimethylsiloxysilicic acid | 3 | 3 | 3 |
| Polyoxybutylene (9) polyoxypropylene (1) glycol | 2 | 2 | 2 |
| Isostearic acid | 0.5 | 0.5 | 0.5 |
| Diisopropyl sebacate | 5 | 5 | 5 |
| Isopropyl myristate | 3 | - | - |
| Glyceryl tri-2-ethylhexanoate | - | 5 | 5 |
| Cetyl 2-ethylhexanoate | - | 5 | 5 |
| Methyl polysiloxane | 3.5 | 3.5 | 3.5 |
| Dimethyl distearyl ammonium hectorite | 0.5 | 0.5 | 0.5 |
| Decamethylcyclopentasiloxane | 13.05 | 13.05 | 13.05 |
| Lauryl PEG-9 polydimethylsiloxyethyl dimethicone | 1 | 1 | 1 |
| Crosslinked poly(methyl methacrylate) (average particle size 8 μm) | 5 | 5 | 5 |
| Crosslinked silicone-network silicone block copolymer (average particle size 5 μm) | 10 | 10 | 10 |
| Purified water | bal | bal | bal |
| Table salt | s.a. | s.a. | s.a. |
| Trisodium edetate | s.a. | s.a. | s.a. |
| Glycerin | 1 | 1 | 1 |
| Xylitol | 1 | 1 | 1 |
| Alcohol (*1) | 10 | 10 | 10 |
| In-vitro SPF | 18.8 | 36.1 | 107.5 |
| (*1) Synthetic alcohol 95% (Japan Alcohol Trading Co., Ltd.) | | | |

As shown in Table 3, sufficient improvement of the ultraviolet protection effects was not observed when the blended amount of the (B) non-volatile polar oil was less than 15% by mass, even when the composition of the oil was changed (Comparative Example 4). In contrast therewith, sufficient improvement of the ultraviolet protection effects was observed when the blended amount of the (B) non-volatile polar oil was 15% by mass or more (Examples 9 and 10). In particular, it was confirmed that the ultraviolet protection effects are markedly improved by using a mixture of hydrogenated palm oil, palm oil, palm kernel oil and an organically modified clay mineral as the composition of the (C) oil phase thickener (Example 10).

(Examples 11 to 23)

Water-in-oil emulsion cosmetics having the compositions indicated in Table 4 and Table 5 below were prepared, and the cumulative absorbance (Abs) values were measured in the same manner as above.

[Table 4]

| | Ex. 11 | Ex. 12 | Ex. 13 | Ex. 14 | Ex. 15 | Ex. 16 | Ex. 17 |
|---|---|---|---|---|---|---|---|
| Purified water | bal | bal | bal | bal | bal | bal | bal |
| 2-Ethylhexyl para-methoxycinnamate | 8 | 8 | 8 | 8 | 8 | 8 | 8 |
| Diethylamino hydroxybenzoyl hexyl benzoate | 2 | 2 | 2 | 2 | 2 | 2 | 2 |

(continued)

| | Ex. 11 | Ex. 12 | Ex. 13 | Ex. 14 | Ex. 15 | Ex. 16 | Ex. 17 |
|---|---|---|---|---|---|---|---|
| Dextrin palmitate | 3 | 3 | 3 | 3 | 3 | 3 | 3 |
| Cetyl 2-ethylhexanoate | 5 | 5 | 5 | 5 | 5 | 5 | 5 |
| Glyceryl tri-2-ethylhexanoate | 5 | 5 | 5 | 5 | 5 | 5 | 5 |
| Diisopropyl sebacate | 5 | 5 | 5 | 5 | 5 | 5 | 5 |
| Silicone KF96-A-6T | 2 | 2 | 2 | 2 | 2 | 2 | 2 |
| Decamethylcyclopentasiloxane | 30 | 30 | 30 | 30 | 30 | 30 | 30 |
| Lauryl PEG-9 polydimethylsiloxyethyl dimethicone | 3 | 3 | 3 | 3 | 3 | 3 | 3 |
| Decamethyltetrasiloxane | 10 | 10 | 10 | 10 | 10 | 10 | 10 |
| Dimethyl distearyl ammonium hectorite | 0.5 | 0.5 | 0.5 | 0.5 | 0.5 | 0.5 | 0.5 |
| Alcohol (*1) | 10 | 10 | 10 | 10 | 10 | 10 | 10 |
| Glycerin | 1 | 1 | 1 | 1 | 1 | 1 | 1 |
| Trisodium edetate | s.a. | s.a. | s.a. | s.a. | s.a. | s.a. | s.a. |
| Fragrance | s.a. | s.a. | s.a. | s.a. | s.a. | s.a. | s.a. |
| Table salt | s.a. | s.a. | s.a. | s.a. | s.a. | s.a. | s.a. |
| Crosslinked silicone-network silicone block copolymer (30 μm) | - | 10 | - | - | - | - | - |
| Calcium stearate-treated talc (average particle size 7 μm) | - | - | - | 10 | - | - | - |
| Crosslinked silicone-network silicone block copolymer (5 μm) | - | - | - | - | 10 | - | - |
| Methylsiloxane network polymer (average particle size 6 μm) | - | - | - | - | - | 10 | - |
| Silicic anhydride (average particle size 5 μm) | - | - | - | - | - | - | 10 |
| Hydrophobically treated zinc oxide | - | - | 10 | - | - | - | - |
| Cumulative absorbance (Abs) | 182.1 | 145.4 | 202.2 | 226.8 | 220.2 | 194.4 | 232.7 |
| (*1) Synthetic alcohol 95% (Japan Alcohol Trading Co., Ltd.) | | | | | | | |

[Table 5]

| | Ex. 18 | Ex. 19 | Ex. 20 | Ex. 21 | Ex. 22 | Ex. 23 |
|---|---|---|---|---|---|---|
| Purified water | bal | bal | bal | bal | bal | bal |
| 2-Ethylhexyl para-methoxycinnamate | 8 | 8 | 8 | 8 | 8 | 8 |
| Diethylamino hydroxybenzoyl hexyl benzoate | 2 | 2 | 2 | 2 | 2 | 2 |
| Dextrin palmitate | 3 | 3 | 3 | 3 | 3 | 3 |
| Cetyl 2-ethylhexanoate | 5 | 5 | 5 | 5 | 5 | 5 |
| Glyceryl tri-2-ethylhexanoate | 5 | 5 | 5 | 5 | 5 | 5 |
| Diisopropyl sebacate | 5 | 5 | 5 | 5 | 5 | 5 |
| Silicone KF96-A-6T | 2 | 2 | 2 | 2 | 2 | 2 |
| Decamethylcyclopentasiloxane | 30 | 30 | 30 | 30 | 30 | 30 |

(continued)

| | Ex. 18 | Ex. 19 | Ex. 20 | Ex. 21 | Ex. 22 | Ex. 23 |
|---|---|---|---|---|---|---|
| Lauryl PEG-9 polydimethylsiloxyethyl dimethicone | 3 | 3 | 3 | 3 | 3 | 3 |
| Decamethyltetrasiloxane | 10 | 10 | 10 | 10 | 10 | 10 |
| Dimethyl distearyl ammonium hectorite | 0.5 | 0.5 | 0.5 | 0.5 | 0.5 | 0.5 |
| Alcohol (*1) | 10 | 10 | 10 | 10 | 10 | 10 |
| Glycerin | 1 | 1 | 1 | 1 | 1 | 1 |
| Trisodium edetate | s.a. | s.a. | s.a. | s.a. | s.a. | s.a. |
| Fragrance | s.a. | s.a. | s.a. | s.a. | s.a. | s.a. |
| Table salt | s.a. | s.a. | s.a. | s.a. | s.a. | s.a. |
| Calcium stearate-treated talc (average particle size 22 μm) | 10 | - | - | - | - | - |
| Dimethylpolysiloxane-treated talc (average particle size 12μm) | - | 10 | - | - | - | - |
| Perfluorooctyltriethoxysilane/alkyl acrylate copolymer methyl polysiloxane ester-treated talc (average particle size 12 μm) | - | - | 10 | - | - | - |
| Carboxydecyltrisiloxane zinc salt-treated talc (average particle size 12 μm) | - | - | - | 10 | - | - |
| Triethoxysilylethyl polydimethylsiloxyethyl hexyl dimethicone-treated talc (average particle size 12 μm) | - | - | - | - | 10 | - |
| Triethoxysilylethyl polydimethylsiloxyethyl hexyl dimethicone-treated talc (average particle size 7 μm) | - | - | - | - | - | 10 |
| Cumulative absorbance (Abs) | 143.1 | 189.6 | 195.4 | 189.1 | 194.3 | 218.7 |
| (*1) Synthetic alcohol 95% (Japan Alcohol Trading Co., Ltd.) | | | | | | |

As shown in Table 4 and Table 5, by adding (D) a powder having an average particle size of 15 μm or smaller, the ultraviolet protection effects were further improved, and it was confirmed that the ultraviolet protection effects were raised to a level equivalent to or even higher than that when an ultraviolet scattering agent was blended (Example 13). Additionally, it was confirmed that hydrophobically treated talc improves the ultraviolet protection effects, regardless of the type of hydrophobic treatment (Examples 14 and 19 to 23). On the other hand, it was confirmed that, when a powder having an average particle size larger than 15 μm was blended, sufficiently high ultraviolet protection effects were exhibited, but there were cases (Examples 12 and 18) in which the ultraviolet protection effects were conversely reduced in comparison to those before the powder was blended (Example 11).

Hereinafter, formulation examples of the water-in-oil emulsion cosmetic of the present invention will be described. Needless to say, the present invention is not in any way limited by these formulation examples, and is rather defined by the claims. The blended amounts are all indicated in % by mass relative to the overall amount of the water-in-oil emulsion cosmetic.

Formulation Example 1. W/O-type BB cream

| (Component Name) | Blended amount (% by mass) |
|---|---|
| Purified water | balance |
| Alcohol | 5 |
| Trisodium edetate | 0.1 |
| Table salt | 0.1 |
| Sodium pyrosulfite | 0.01 |
| Phenoxyethanol | 1 |

(continued)

| (Component Name) | Blended amount (% by mass) |
|---|---|
| Glycerin | 5 |
| Erythritol | 1 |
| Xylitol | 1 |
| Tormentilla extract | 0.1 |
| Sodium hyaluronate | 0.1 |
| 2-O-ethyl L-ascorbic acid | 0.1 |
| Dipotassium glycyrrhizinate | 0.05 |
| Isopropyl myristate | 5 |
| Glyceryl tri-2-ethylhexanoate | 5 |
| Diisopropyl sebacate | 5 |
| Alkyl ($C_{12-15}$) benzoate | 3 |
| Methyl polysiloxane | 6 |
| Cyclopentasiloxane | 6 |
| 50% Trisiloxysilicic acid in cyclopentasiloxane solution | 2 |
| Dextrin palmitate | 2 |
| 2-Ethylhexyl methoxycinnamate | 5 |
| Homosalate | 5 |
| Hydrophobically treated fine-particle titanium oxide (particle size 15 nm) | 3 |
| Hydrophobically treated fine-particle zinc oxide (particle size 15 nm) | 3 |
| Hydrophobically treated pigment-grade titanium oxide | 3 |
| Hydrophobically treated red iron oxide | s.a. |
| Hydrophobically treated yellow iron oxide | s.a. |
| Hydrophobically treated black iron oxide | s.a. |
| Hydrophobically treated talc | 3 |
| Crosslinked silicone-network silicone block copolymer (average particle size 5 $\mu$m) | 6 |
| Fine-particle dimethylsilylated silica | 0.5 |
| Lauryl PEG-9 polydimethylpolysiloxyethyl dimethicone | 2 |
| Dimethyl distearyl ammonium hectorite | 1 |
| Dextrin palmitate | 0.5 |
| Isostearic acid | 0.2 |
| Tocopherol | 0.01 |
| Fragrance | s.a. |

Formulation Example 2. W/O-type base cosmetic

| (Component Name) | Blended amount (% by mass) |
|---|---|
| Purified water | balance |
| Alcohol | 10 |
| Trisodium edetate | 0.1 |
| Table salt | 0.1 |
| Sodium pyrosulfite | 0.01 |
| Glycerin | 1 |
| Xylitol | 1 |
| Tormentilla extract | 0.1 |
| Sodium hyaluronate | 0.1 |
| 2-O-ethyl L-ascorbic acid | 0.1 |
| Dipotassium glycyrrhizinate | 0.05 |
| Isododecane | 5 |

(continued)

| (Component Name) | Blended amount (% by mass) |
|---|---|
| Diisopropyl sebacate | 8 |
| Glyceryl tri-2-ethylhexanoate | 5 |
| Isopropyl myristate | 5 |
| PBG/PPG-9/1 copolymer | 2 |
| Methyl polysiloxane | 5 |
| Cyclopentasiloxane | 3 |
| Caprylyl methicone | 3 |
| 20% Highly polymerized aminopropyl dimethicone in dimethicone 20 cs solution | 1 |
| 50% Trifluoroalkyl dimethyl trimethylsiloxysilicic acid dimethicone solution | 3 |
| Dextrin palmitate | 2 |
| 2-Ethylhexyl methoxycinnamate | 5 |
| Diethylamino hydroxybenzoyl hexyl benzoate | 1 |
| bis-Ethylhexyloxyphenol methoxyphenyl triazine | 0.5 |
| Hydrophobically treated fine-particle titanium oxide (particle size 15 nm) | 2 |
| Hydrophobically treated fine-particle zinc oxide (particle size 15 nm) | 5 |
| Hydrophobically treated pigment-grade titanium oxide | 5 |
| Hydrophobically treated red iron oxide | s.a. |
| Hydrophobically treated yellow iron oxide | s.a. |
| Hydrophobically treated black iron oxide | s.a. |
| Hydrophobically treated talc | 5 |
| Crosslinked silicone-network silicone block copolymer (average particle size 5 μm) | 2 |
| Hydrophobically treated talc | 3 |
| PEG-9 polydimethylpolysiloxyethyl dimethicone | 1.5 |
| 50% PEG/PPG-19/19 dimethicone in cyclopentasiloxane solution | 0.5 |
| Dimethyl distearyl ammonium hectorite | 0.4 |
| Isostearic acid | 0.3 |
| Tocopherol | 0.01 |
| Fragrance | s.a. |

Formulation Example 3. W/O-type hair cosmetic

| (Component Name) | Blended amount (% by mass) |
|---|---|
| Purified water | balance |
| Alcohol | 8 |
| Trisodium edetate | 0.2 |
| Silica | 0.5 |
| Glycerin | 1 |
| Polyoxyethylene (14) polyoxypropylene (7) dimethyl ether | 1 |
| *Rosa canina* fruit oil | 0.1 |
| Sodium hyaluronate | 0.1 |
| 2-O-ethyl L-ascorbic acid | 0.5 |
| Dipotassium glycyrrhizinate | 0.05 |
| Isododecane | 10 |
| Glyceryl tri-2-ethylhexanoate | 5 |
| Isopropyl myristate | 5 |
| Diisopropyl sebacate | 5 |
| Alkyl ($C_{12-15}$) benzoate | 3 |
| PBG/PPG-9/1 copolymer | 1 |

(continued)

| (Component Name) | Blended amount (% by mass) |
|---|---|
| Methyl polysiloxane | 10 |
| Cyclopentasiloxane | 3 |
| 50% Trisiloxysilicic acid in cyclopentasiloxane solution | 0.5 |
| Sucrose tetrastearate triacetate | 1 |
| Dextrin palmitate | 2 |
| 2-Ethylhexyl methoxycinnamate | 5 |
| Diethylamino hydroxybenzoyl hexyl benzoate | 2 |
| bis-Ethylhexyloxyphenol methoxyphenyl triazine | 1 |
| Polysilicone-15 | 3 |
| Octocrylene | 5 |
| Crosslinked silicone-network silicone block copolymer (average particle size 5 μm) | 10 |
| Calcium stearate-treated talc (average particle size 7 μm) | 3 |
| Cetyl PEG/PPG-10/1 dimethicone | 1 |
| Lauryl PEG-9 polydimethylpolysiloxyethyl dimethicone | 1 |
| Dimethyl distearyl ammonium hectorite | 0.5 |
| Isostearic acid | 0.3 |
| Sorbitan sesquiisotearate | 0.3 |
| Tocopherol | 0.01 |
| Fragrance | s.a. |

## Claims

1. A water-in-oil emulsion cosmetic containing:

   (A) 6 to 40% by mass of an ultraviolet absorbing agent;
   (B) 15% by mass or more of a non-volatile polar oil having ester bonds (except that the (A) ultraviolet absorbing agent is not included); and
   (C) 0.5 to 15% by mass of an oil phase thickener.

2. The water-in-oil emulsion cosmetic as in claim 1, wherein the (C) oil phase thickener is of one or more types selected from among dextrin fatty acid esters, sucrose fatty acid esters, fatty acids or salts thereof, hardened vegetable oils, solid or semi-solid vegetable oils, organically modified clay minerals, glyceryl fatty acid esters and amino acid-based gelling agents.

3. The water-in-oil emulsion cosmetic as in claim 2, wherein two or more types of the (C) oil phase thickener are blended, one of the types being an organically modified clay mineral.

4. The water-in-oil emulsion cosmetic as in claim 3, wherein a blended amount of a (C) oil phase thickener other than the organically modified clay mineral is 2% by mass or more.

5. The water-in-oil emulsion cosmetic as in any one of claims 1 to 4, wherein the (A) ultraviolet absorbing agent includes at least one type selected from among ethylhexyl methoxycinnamate, octocrylene, 2-hydroxy-4-methoxybenzophenone, dimethicodiethylbenzalmalonate, t-butyl methoxydibenzoylmethane, ethylhexyl triazone, diethylamino hydroxybenzoyl hexyl benzoate, bis-ethylhexyloxyphenol methoxyphenyl triazine, methylene bis-benzotriazolyl tetramethylbutylphenol, phenylbenzimidazole sulfonic acid, homosalate and ethylhexyl salicylate.

6. The water-in-oil emulsion cosmetic as in any one of claims 1 to 5, further containing (D) a powder having an average particle size of 15 μm or smaller, the powder being of one or more types selected from among (i) talc, (ii) silicone powder, (iii) crosslinked poly(methyl methacrylate) powder, and (iv) silica.

7. The water-in-oil emulsion cosmetic as in claim 6, wherein the (D) powder is hydrophobically treated talc.

**INTERNATIONAL SEARCH REPORT**

International application No.
PCT/JP2018/021662

A. CLASSIFICATION OF SUBJECT MATTER
Int.Cl. A61K8/37(2006.01)i, A61K8/06(2006.01)i, A61K8/25(2006.01)i, A61K8/35(2006.01)i, A61K8/36(2006.01)i, A61K8/40(2006.01)i, A61K8/41(2006.01)i, A61K8/60(2006.01)i, A61K8/73(2006.01)i, A61K8/81(2006.01)i, A61K8/895(2006.01)i, A61K8/92(2006.01)i, A61Q17/04(2006.01)i
According to International Patent Classification (IPC) or to both national classification and IPC

B. FIELDS SEARCHED
Minimum documentation searched (classification system followed by classification symbols)
Int.Cl. A61K8/00-99, A61Q1/00-9/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y | JP 2013-63954 A (SHISEIDO CO., LTD.) 11 April 2013, paragraphs [0022]-[0044] & US 2013/0344013 A1, paragraphs [0037]-[0078] & WO 2013/031510 A1 & EP 2692338 A1 & CN 103501759 A | 1-2, 5<br>3-7 |
| X<br>Y | JP 2011-126832 A (SHISEIDO CO., LTD.) 30 June 2011, paragraphs [0007], [0034], [0041] (Family: none) | 1-5<br>3-7 |
| Y | JP 2003-119111 A (TOKIWA CO., LTD.) 23 April 2003, paragraphs [0022], [0029] (Family: none) | 6-7 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 29 June 2018 (29.06.2018) | 10 July 2018 (10.07.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

INTERNATIONAL SEARCH REPORT

International application No.
PCT/JP2018/021662

C (Continuation). DOCUMENTS CONSIDERED TO BE RELEVANT

| Category* | Citation of document, with indication, where appropriate, of the relevant passages | Relevant to claim No. |
|---|---|---|
| Y | WO 2017/057675 A1 (SHISEIDO CO., LTD.) 06 April 2017, paragraphs [0019], [0025], [0058] & JP 2017-66137 A | 7 |
| Y | JP 2001-58934 A (SHISEIDO CO., LTD.) 06 March 2001, paragraphs [0018], [0029], [0033] & US 6749838 B1, column 7, lines 36-50, column 10, lines 10-45, column 11, lines 5-42 & WO 2001/013874 A1 & EP 1123697 A1 & CN 1320031 A | 7 |
| Y | JP 10-175819 A (NOEVIR CO., LTD.) 30 June 1998, paragraphs [0028]-[0029], [0035]-[0039] (Family: none) | 7 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2011126832 A


### Non-patent literature cited in the description


- Shin-keshohin-gaku [New Cosmetology. Nanzando, 2001, 497-504